# EUROPEAN PATENT SPECIFICATION

(11) **EP 3 766 462 B1**
(45) Date of publication and mention of the grant of the patent: **26.10.2022**
(21) Application number: 19840301.6
(22) Date of filing: 11.04.2019
(51) Int. Cl.: A61F 2/95, A61M 27/00, A61F 2/94

(54) **TUBE STENT DELIVERY SYSTEM**
SYSTEM ZUR RÖHRCHENSTENTEINFÜHRUNG
SYSTÈME DE POSE DE STENT TUBULAIRE

(30) Priority: 25.07.2018 JP 2018138996
(43) Date of publication of application: 20.01.2021
(73) Proprietor: Silux Co., Ltd., Kawaguchi-shi, Saitama 333-0816 (JP)
(72) Inventor: FURUHASHI, Shinichi, Kawaguchi-shi, Saitama 333-0816 (JP)
(74) Representative: Lichtnecker, Markus Christoph
(86) International application number: PCT/JP2019/015842
(87) International publication number: WO 2020/021781

(56) References cited:
- DE-A1-102007 038 669
- JP-A- H09 256 633
- JP-A- 2008 012 307
- JP-A- 2013 540 970
- JP-A- 2015 073 547
- JP-A- 2015 073 547
- JP-A- 2015 181 875
- US-A1- 2011 077 622
- US-A1- 2012 095 567
- US-A1- 2012 095 567

## Description

### Technical Field

The present invention relates to a tube stent delivery system used for biliary drainage, pancreatic duct drainage, and the like.

### Background Art

There are some tube stent delivery systems capable of pulling back a tube stent when the tube stent is carried into the body and failed in desired positioning (refer to PTLs 1 and 2).

There is, for example, a tube stent delivery system (hereinafter, referred to as a conventional tube stent delivery system) in which a tube stent and a pusher catheter are coupled via a thread and withdrawal of the inner catheter allows removal of the coupling of the tube stent with the pusher catheter.

PTL 1 also discloses a stent delivery system having a pusher catheter with a hole to attach a filament at an end portion and a stent with a latch hole, wherein an inner catheter maintains a state of loosely fitting a nodule to the latch hole from inside the stent, causing the state of coupling the stent with the pusher catheter to be less likely to be accidentally removed until the inner catheter is withdrawn and withdrawal of the inner catheter allows easy removal of the state of coupling the stent with the pusher catheter.

PTL 2 discloses a tube stent delivery device, including: an inner catheter with a tube stent axially movably mounted on a distal end outer circumference; and an outer catheter axially movably mounted on an outer circumference of the inner catheter located on a proximal end side of the tube stent, wherein an intermediate stopper with an outer diameter greater than an inner diameter of a distal end opening of the tube stent is formed in a predetermined position on the proximal end side from the distal end of the inner catheter, facilitating adjustment of the position of the tube stent and allowing secure indwelling of the tube stent in an accurate position in the body without entanglement of a thread or a cord in the body.

PTL 3 discloses a tube stent delivery system according to the preamble of claim 1.

PTL 4 and PTL 5 disclose tube stent delivery systems without a second fitting hole or a second fitting piece.

### Citation List

### Patent Literature

PTL 1: JP 2012-239803 A
PTL 2: JP 2015-36043 A
PTL 3: US 2011/077622 A1
PTL 4: US 2012/095567 A1
PTL 5: JP 2015 073547 A

### Summary of Invention

### Technical Problem

However, such a conventional stent delivery system holds the tube stent by tying up with a thread. The thread is thus tightened at a tube stent connecting section by strongly pulling the pusher catheter (outer catheter) in the endoscope in a combined state or withdrawing the inner catheter while the wire is remaining and then pulling the pusher catheter. As a result, there is a problem that the thread cuts into the tube stent (the stent is damaged) and removal (release) becomes difficult.

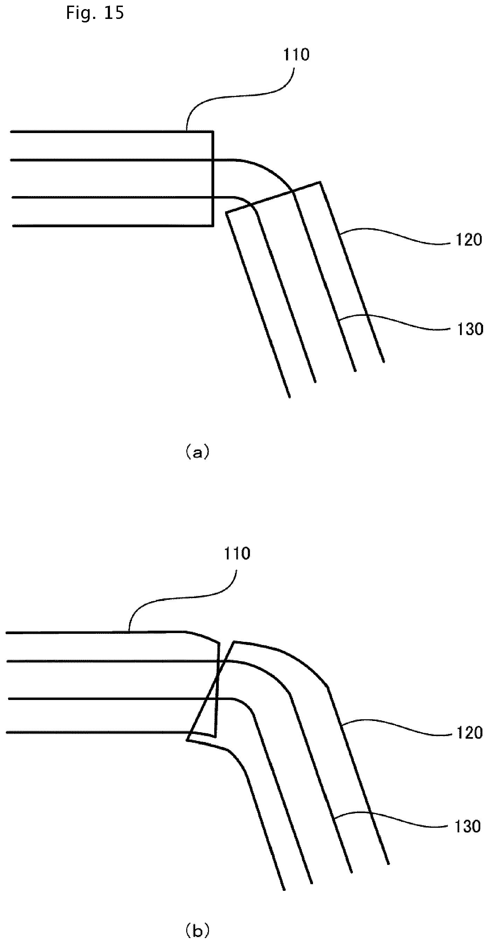

As illustrated in Figs. 15, release action in the conventional stent delivery system and the stent delivery system in PTL 1 while the axis of a tube stent 110 does not coincide with the axis of a pusher catheter (outer catheter 120) and the axes are angled (refer to Fig. 15A) sometimes causes incorrect contact of the distal end portion of the pusher catheter with the rear end portion of the tube stent and thus causes the rear end portion of the tube stent to cut into the distal end portion of the pusher catheter (refer to Fig. 15B). This results in a problem of causing a defect in release (removal) due to high resistance on the inner tube (inner catheter) when the inner tube is withdrawn from the tube stent.

The stent delivery system in PTL 1 also has a problem that the tube stent is held only by a knot of the thread loosely fit to the latch hole and thus the thread knot is likely to be untied, leading to a possibility of unintended stent fall out.

The tube stent delivery device in PTL 2 is different from the conventional stent delivery system described above and the stent delivery system in PTL 1 in that no thread is used for combination of the pusher catheter with the tube stent, and thus entanglement of a thread does not occur in the body or the endoscope.

This tube stent delivery device, however, sometimes varies in resistance during withdrawal of the inner tube from the tube stent depending on the machining precision of the tube stent and the inner tube. This results in a problem of causing a possibility of false release of the tube stent during operation.

The tube stent delivery device has to be provided with the tube stent and the inner tube having an area with a partially different diameter, such as an intermediate stopper, with machining precision of 10⁻² mm. The device thus has a problem of taking time for such machining.

The tube stent delivery device has to have the resistance during withdrawal of the inner tube from the tube stent falling within a predetermined range. This results in a problem of taking time to adjust the resistance between the tube stent and the inner tube.

The tube stent delivery systems and the tube stent delivery device described above have a problem of not allowing manufacture of those with an outer diameter of 1.67 mm (5 French gauge) or less due to the issues of machining precision and material strength.

In view of the above circumstances, it is an object of the present invention to provide a tube stent delivery system having a simple structure and allowed to be smaller in size and readily release a tube stent.

### Solution to Problem

Since the conventional tube stent delivery system has to insert the tube stent, a pusher catheter, and the like into a narrow area, such as the duodenum, there was no idea that the axis of the tube stent and the axis of the pusher catheter are positively shift when the tube stent is released.

As a result of intensive research and development on the problems described above, the inventor of the present invention has found a breakthrough tube stent delivery system as below that defeats the situation without such an idea.

A tube stent delivery system of the present invention includes: a tube stent; an outer catheter; and an inner catheter, wherein the tube stent has a first tube wall section in an annular shape, a hollow first insertion section surrounded by the first tube wall section, and a first fitting section fitted to the outer catheter, the first fitting section has a first fitting piece, a first fitting hole, and a first body, the outer catheter has a second tube wall section in an annular shape, a hollow second insertion section surrounded by the second tube wall section, and a second fitting section fitted to the tube stent, the second fitting section has a second fitting piece, a second fitting hole, and a second body, the first fitting piece is inserted into the second fitting hole and the second fitting piece is inserted into the first fitting hole, thereby performing the fitting between the first fitting section and the second fitting section, the inner catheter is inserted into the first insertion section of the tube stent and the second insertion section of the outer catheter, thereby maintaining the fitting, such that when the inner catheter is withdrawn from at least the first insertion section and the first fitting section of the tube stent, the fitting is separated.

### Advantageous Effects of Invention

According to the tube stent delivery system of the present invention, junction of the tube stent with the outer catheter is securely formed by fitting of the fitting sections to each other and insertion of the inner catheter into the insertion section makes the fitting firmer, thereby allowing secure delivery of the tube stent to a target site in the body without separation and also allowing easy disconnection of the fitting only by withdrawing the inner catheter to immediately indwell the tube stent at the target site. The tube stent delivery system of the present invention also allows stable operation of indwelling positioning by pulling the tube stent. The tube stent delivery system of the present invention further allows providing a tube stent delivery system with a narrow diameter that has not been manufacturable because the system has no excessive change in diameter and thread.

### Brief Description of the Drawings

Fig. 1 is a side view illustrating an example of a tube stent delivery system according to the present invention.
Figs. 2 illustrate a tube stent in Fig. 1, where Fig. 2A is a side view and Fig. 2B is a cross-sectional view along A-A of Fig. 2A.
Figs. 3 illustrate an outer catheter in Fig. 1, where Fig. 3A is a side view and Fig. 3B is a cross-sectional view along A-A of Fig. 3A.
Fig. 4 is a side view illustrating the inner catheter in Fig. 1.
Figs. 5 illustrate an example of a first embodiment of fitting sections according to the present invention, where Fig. 5A is a cross-sectional view of a first fitting section, Fig. 5B is a cross-sectional view of a second fitting section, and Fig. 5C is a cross-sectional perspective view illustrating a fitting state of the first fitting section and the second fitting section.
Figs. 6 illustrate the example following Figs. 5, where Fig. 6A is a side view illustrating the fitting state of the first fitting section and the second fitting section and Fig. 6B is a cross-sectional view illustrating a state of inserting an inner catheter in the first fitting section and the second fitting section.

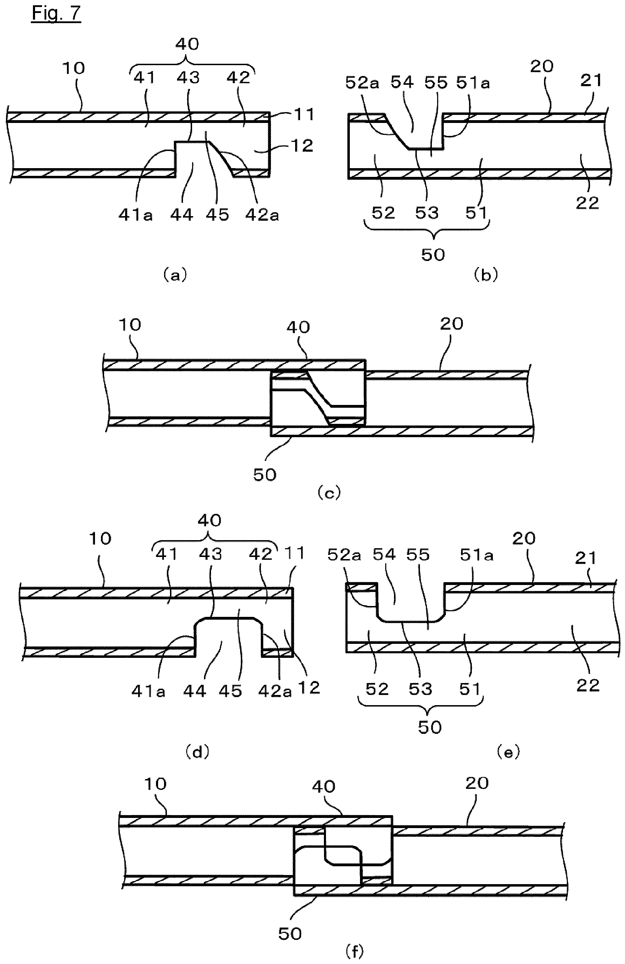

Figs. 7 illustrate fitting sections according to the present invention, where Fig. 7A is a cross-sectional view of a first fitting section in a second embodiment, Fig. 7B is a cross-sectional view of a second fitting section in the second embodiment, Fig. 7C is a cross-sectional perspective view illustrating a fitting state of the first fitting section and the second fitting section in the second embodiment, Fig. 7D is a cross-sectional view of a first fitting section in a third embodiment, Fig. 7E is a cross-sectional view of a second fitting section in the third embodiment, and Fig. 7F is a cross-sectional perspective view illustrating a fitting state of the first fitting section and the second fitting section in the third embodiment.

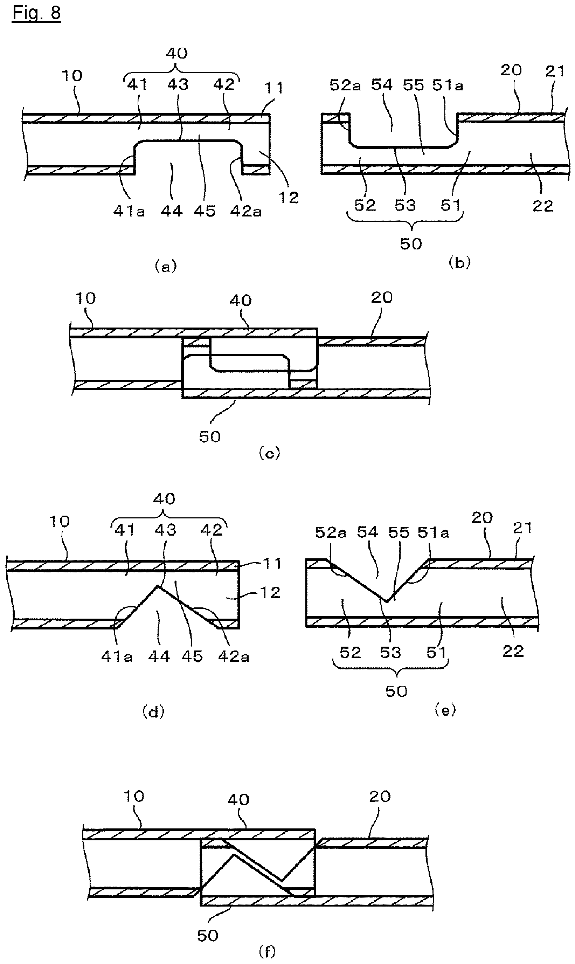

Figs. 8 illustrate fitting sections according to the present invention, where Fig. 8A is a cross-sectional view of a first fitting section in a fourth embodiment, Fig. 8B is a cross-sectional view of a second fitting section in the fourth embodiment, Fig. 8C is a cross-sectional perspective view illustrating a fitting state of the first fitting section and the second fitting section in the fourth embodiment, Fig. 8D is a cross-sectional view of a first fitting section in a fifth embodiment, Fig. 8E is a cross-sectional view of a second fitting section in the fifth embodiment, and Fig. 8F is a cross-sectional perspective view illustrating a fitting state of the first fitting section and the second fitting section in the fifth embodiment.

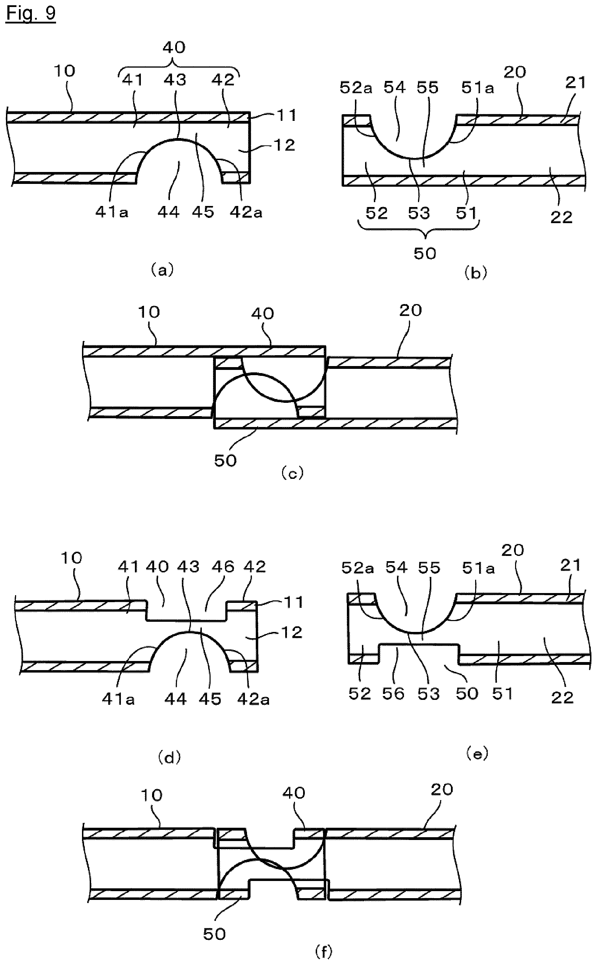

Figs. 9 illustrate fitting sections according to the present invention, where Fig. 9A is a cross-sectional view of a first fitting section in a sixth embodiment, Fig. 9B is a cross-sectional view of a second fitting section in the sixth embodiment, Fig. 9C is a cross-sectional perspective view illustrating a fitting state of the first fitting section and the second fitting section in the sixth embodiment, Fig. 9D is a cross-sectional view of a first fitting section in a seventh embodiment, Fig. 9E is a cross-sectional view of a second fitting section in the seventh embodiment, and Fig. 9F is a cross-sectional perspective view illustrating a fitting state of the first fitting section and the second fitting section in the seventh embodiment.

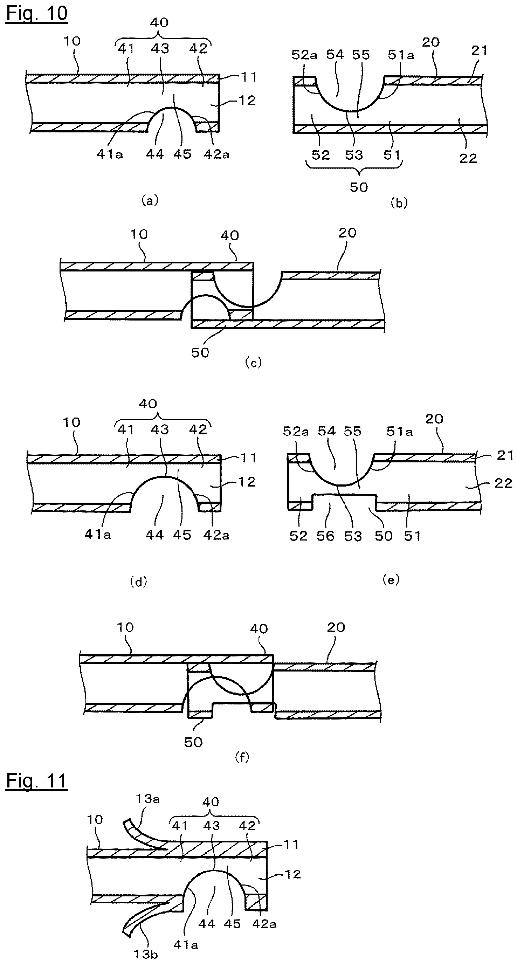

Figs. 10 illustrate fitting sections according to the present invention, where Fig. 10A is a cross-sectional view of a first fitting section in an eighth embodiment, Fig. 10B is a cross-sectional view of a second fitting section in the eighth embodiment, Fig. 10C is a cross-sectional perspective view illustrating a fitting state of the first fitting section and the second fitting section in the eighth embodiment, Fig. 10D is a cross-sectional view of a first fitting section in a ninth embodiment, Fig. 10E is a cross-sectional view of a second fitting section in the ninth embodiment, and Fig. 10F is a cross-sectional perspective view illustrating a fitting state of the first fitting section and the second fitting section in the ninth embodiment.
Fig. 11 is a cross-sectional view of a first fitting section in a tenth embodiment according to the present invention.
Fig. 12 is a schematic diagram illustrating a state of using the tube stent delivery system according to the present invention.
Figs. 13 are explanatory diagrams illustrating a simulation of the tube stent delivery system according to the present invention, where Fig. 13A illustrates a fitting state and Fig. 13B illustrates a separated state.
Fig. 14 is a cross-sectional view of a first fitting section according to the present invention.
Figs. 15 are perspective views of a state where the axis of the tube stent and the axis of the pusher catheter are angled in the conventional stent delivery system.

### Description of Embodiments

Embodiments of a tube stent delivery system according to the present invention are described below with reference to the attached drawings. It should be noted that the present invention is not limited to the following embodiments.

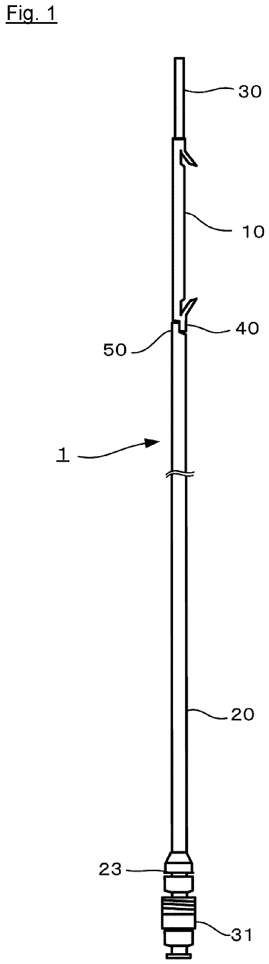

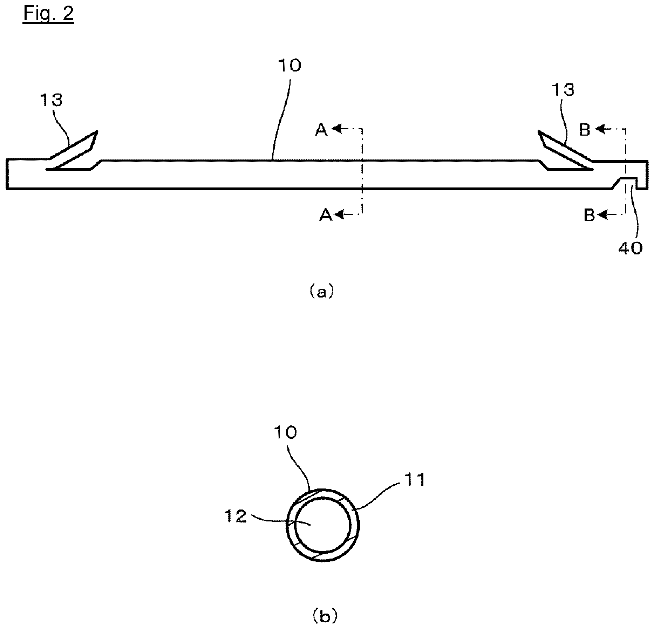

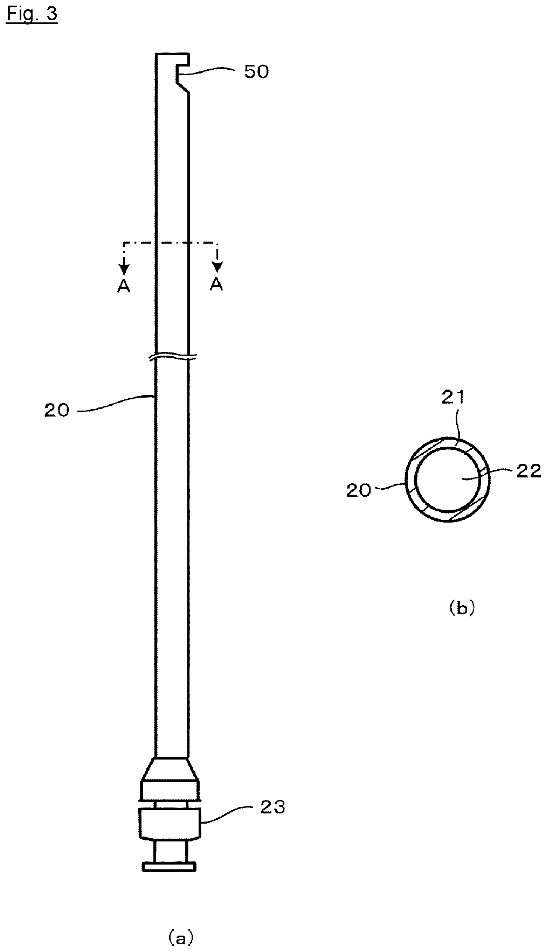

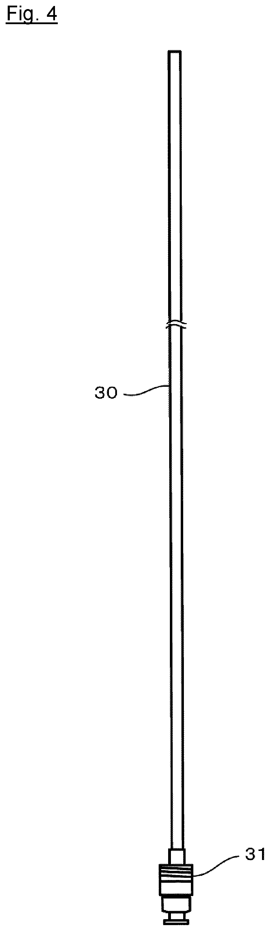

Fig. 1 is a side view illustrating an example of a tube stent delivery system according to the present invention. Figs. 2 illustrate a tube stent in Fig. 1, where Fig. 2A is a side view and Fig. 2B is a cross-sectional view along A-A of Fig. 2A. Figs. 3 illustrate an outer catheter in Fig. 1, where Fig. 3A is a side view and Fig. 3B is a cross-sectional view along A-A of Fig. 3A. Fig. 4 is a side view illustrating the inner catheter in Fig. 1. With reference to Figs. 1 through 4, a description is given to an example of the tube stent delivery system.

As illustrated in Fig. 1, a tube stent delivery system 1 of the present invention includes a tube stent 10, an outer catheter 20, and an inner catheter 30.

As illustrated in Figs. 2, the tube stent 10 is to indwell at a target site in the body and includes a first tube wall section 11 in an annular shape, a first insertion section 12 which is hollow and surrounded by the first tube wall section 11, flap sections 13 arranged on both sides of the tube stent 10, and a first fitting section 40 provided on a junction side with the outer catheter 20.

As illustrated in Figs. 3, the outer catheter 20 has an end joined with the tube stent 10 and includes a second tube wall section 21 in an annular shape, a second insertion section 22 which is hollow and surrounded by the second tube wall section 21, a second fitting section 50 provided on a junction side with the tube stent 10, and an inner connector 23 connected at the other end on the opposite side of the junction side with the tube stent 10.

As illustrated in Fig. 4, the inner catheter 30 has a rod shape to be inserted into the first insertion section 12 of the tube stent 10 and the second insertion section 22 of the outer catheter 20 and has an end provided with an outer connector 31 to be combined with the inner connector 23.

The tube stent 10, the outer catheter 20, and the inner catheter 30 are formed from a flexible resin material (e.g., polyethylene, polyurethane, polyamide, fluorocarbon polymers (polytetrafluoroethylene, perfluoroalkoxyalkane, an ethylene-tetrafluoroethylene copolymer, a perfluoroethylene-propene copolymer), etc.) and the material is not particularly limited.

The tube stent delivery system 1 allows the tube stent 10, the first fitting section 40, and the second fitting section 50 of the outer catheter 20 to be fit to each other, followed by maintaining the fitting by inserting the inner catheter 30 into the first insertion section 12 of the tube stent 10 and the second insertion section 22 of the outer catheter 20, and removing the fitting by withdrawing the inner catheter 30 from the first insertion section 12 and the second insertion section 22 to separate the tube stent 10 from the outer catheter 20.

The tube stent delivery system 1 of the present invention is a system in which the tube stent 10 is joined with a distal end portion of the outer catheter 20 and delivered to a target site in the body for, for example, biliary or pancreatic drainage. The state where the tube stent 10 is joined with the outer catheter 20 thus has to be maintained to deliver the tube stent 10 to the target site.

In the present invention, such junction is the fitting where the first fitting section 40 and the second fitting section 50 overlap each other. The fitting of the first fitting section 40 and the second fitting section 50 is maintained in the state of inserting the inner catheter 30 into the first insertion section 12 and the second insertion section 22.

The tube stent 10 delivered to the target site in the body is allowed to readily remove the fitting of the first fitting section 40 and the second fitting section 50 by withdrawing the inner catheter from the first fitting section 40 of the first insertion section 12 and the second fitting section 50 of the second insertion section 22, followed by shifting an axis of the tube stent 10 from an axis of the outer catheter 20. As a result, it is possible to indwell the tube stent 10 at the target site in the body.

That is, the tube stent delivery system 1 according to the present invention allows disconnection of the fitting between the tube stent 10 and the outer catheter 20 to readily separate the tube stent 10 from the outer catheter 20 only by the operation of inserting the inner catheter 30 into the tube stent 10 and the outer catheter 20 and withdrawing the inner catheter 30, followed by shifting the axis of the tube stent 10 from the axis of the outer catheter 20.

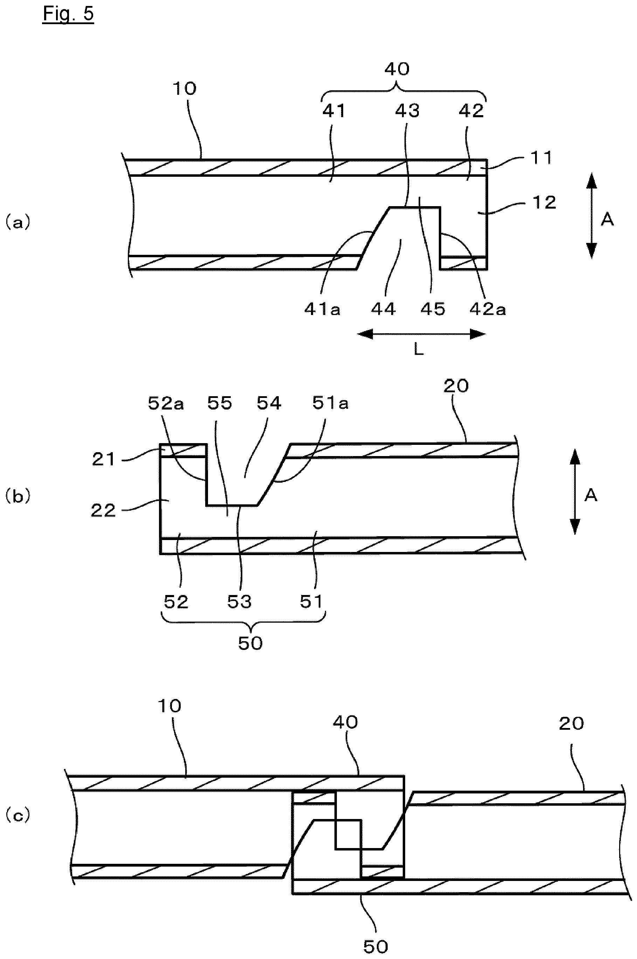

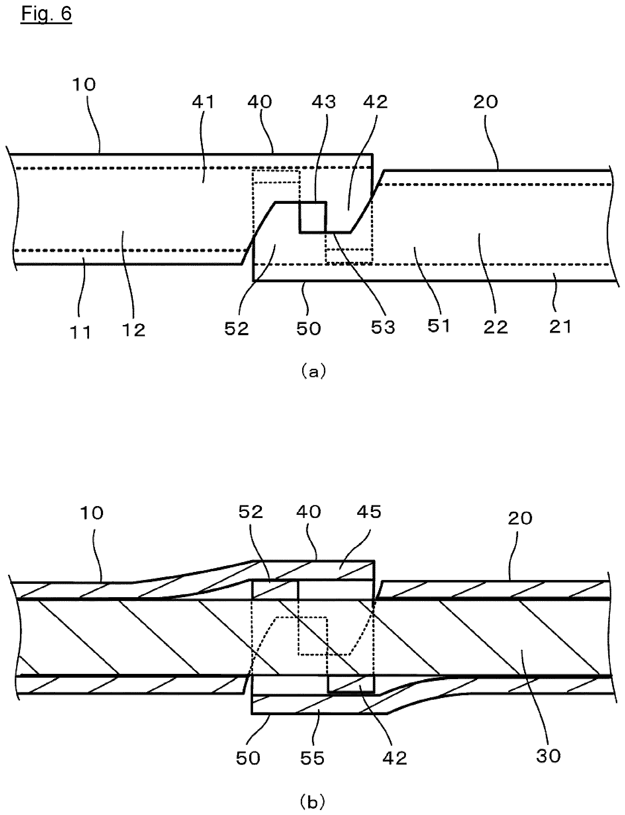

A detailed description is then given to the relationship between the first fitting section 40 and the second fitting section 50 in the tube stent delivery system 1. Figs. 5 illustrate an example of the first embodiment of fitting sections according to the present invention, where Fig. 5A is a cross-sectional view of the first fitting section, Fig. 5B is a cross-sectional view of the second fitting section, and Fig. 5C is a cross-sectional perspective view illustrating the fitting state of the first fitting section and the second fitting section. Figs. 6 illustrate the example following Figs. 5, where Fig. 6A is a side view illustrating the fitting state of the first fitting section and the second fitting section and Fig. 6B is a cross-sectional view illustrating a state of inserting an inner catheter in the first fitting section and the second fitting section. The first fitting section 40 and the second fitting section 50 are described in detail based on Figs. 5 and 6. It should be noted that, although the description is given using the terms such as "the first" and "the second", the terms do not limit the positional relationship and are used to facilitate understanding of the description.

As illustrated in Fig. 5A, the first fitting section 40 of the tube stent 10 includes a first body 41 surrounded by the first tube wall section 11, a first fitting piece 42 with the cross section in an annular shape maintaining the first tube wall section 11 provided at the distal end portion, and a first fitting hole 43 coupling the first body 41 and the first fitting piece 42. The first fitting hole 43 has a first notch portion 44, which is partially notched, and a first coupling section 45 with the partially remaining first tube wall section 11. That is, the first fitting hole 43 is not a completely penetrating through hole but a closed tube hole, which is partially closed.

The first fitting hole 43 is in a state of being open to a direction orthogonal to a longitudinal direction of the tube stent 10. The first fitting hole 43 is configured with a first fitting piece side wall 42a on the first fitting piece 42 side, a first body side wall 41a on the first body 41 side, and the first coupling section 45 integrally linking the first fitting piece side wall 42a to the first body side wall 41a.

Meanwhile, as illustrated in Fig. 5B, the second fitting section 50 of the outer catheter 20 includes a second body 51 surrounded by the second tube wall section 21, a second fitting piece 52 with the cross section in an annular shape maintaining the second tube wall section 21 provided at the distal end portion, and a second fitting hole 53 coupling the second body 51 with the second fitting piece 52. The second fitting hole 53 has a second notch portion 54, which is partially notched, and a second coupling section 55 with the partially remaining second tube wall section 21. That is, the second fitting hole 53 is not a completely penetrating through hole but a closed tube hole, which is partially closed.

The second fitting hole 53 is in a state of being open to a direction orthogonal to a longitudinal direction of the outer catheter 20. The second fitting hole 53 is configured with a second fitting piece side wall 52a on the second fitting piece 52 side, a second body side wall 51a on the second body 51 side, and the second coupling section 55 integrally linking the second fitting piece side wall 52a to the second body side wall 51a.

The first fitting section 40 and the second fitting section 50 have an identical or similar shape or different shapes, and the first fitting section 40 and the second fitting section 50 are fit by inserting the first fitting piece 42 of the first fitting section 40 into the second fitting hole 53 of the second fitting section 50 and inserting the second fitting piece 52 of the second fitting section 50 into the first fitting hole 43 of the first fitting section 40.

That is, the first fitting section 40 and the second fitting section 50 are fit while overlapping each other and moreover the inner catheter 30 is inserted into them, and it is thus possible to securely carry the tube stent 10 to the target site in the body without disconnecting the fitting. The inner catheter 30 is then withdrawn from the first insertion section 12 to the fitting area of the second insertion section 22 to shift the axis of the tube stent 10 from the axis of the outer catheter 20 (in the present embodiment, shift the axis of the tube stent 10 upward or the axis of the outer catheter 20 downward), thereby readily separating the first fitting section 40 from the second fitting section 50 and allowing quick achievement of release of the tube stent 10 from the outer catheter 20.

In this situation, the first fitting piece 42 is formed from the first insertion section 12 with the entire circumference surrounded by the first tube wall section 11 and is thus not allowed to be separated from the second fitting hole 53 unless the inner catheter 30 is removed while the inner catheter 30 is inserted into the first insertion section 12. Similarly, the second fitting piece 52 is formed from the second insertion section 22 with the entire circumference surrounded by the second tube wall section 21 and is thus not allowed to be separated from the first fitting hole 43 unless the inner catheter 30 is removed while the inner catheter 30 is inserted into the second insertion section 22.

While the first fitting section 40 and the second fitting section 50 are fit to each other, they are slightly shifted in a radial direction due to the difference in radial position. That is, an outer surface of the first fitting piece 42 abuts on an inner surface of the second coupling section 55 and an outer surface of the second fitting piece 52 abuts on an inner surface of the first coupling section 45, causing the radial position shift in the fitting sections 40 and 50. When the inner catheter 30 is inserted into the first insertion section 12 and the second insertion section 22, a pressing force by an outer surface of the inner catheter 30 causes a radial bulge in the first fitting section 40 and the second fitting section 50. The bulge causes the inner catheter 30 to be slightly pressure joined and an increase in combining strength between the first fitting section 40 and the second fitting section 50, thereby not easily separating the tube stent 10 from the outer catheter 20 even during movement in the body.

With reference to Figs. 5 through 10, the respective embodiments of the first fitting section 40 and the second fitting section 50 are further described.

Figs. 5 and 6 illustrate the first embodiment of the fitting sections. In the first embodiment, the first fitting piece side wall 42a of the first fitting hole 43 in the first fitting section 40 is flat to a radial direction of the tube stent 10. That is, the first fitting piece side wall 42a is formed to be vertical to an axial direction of the tube stent 10. Meanwhile, the first body side wall 41a is inclined to the radial direction. That is, the first body side wall 41a is formed to be inclined to the axial direction of the tube stent 10. The first coupling section 45 is parallel and flat to the longitudinal direction (axial direction) of the tube stent 10. It should be noted that the radial direction is illustrated by arrows A in the drawings.

Similarly, the second fitting piece side wall 52a of the second fitting hole 53 in the second fitting section 50 is flat to a radial direction of the outer catheter 20. That is, the second fitting piece side wall 52a is formed to be vertically flat to the axial direction of the tube stent 10. Meanwhile, the second body side wall 51a is inclined to the radial direction of the outer catheter 20. That is, the second body side wall 51a is formed to be inclined to an axial direction of the outer catheter 20. The second coupling section 55 is parallel and flat to the longitudinal direction (axial direction) of the outer catheter 20. In this situation, the first coupling section 45 and the second coupling section 55 may be partially inclined to the axial direction of the tube stent 10 and the outer catheter 20.

In the second embodiment of the fitting sections illustrated in Figs. 7A, 7B, and 7C, on the contrary to the first embodiment, the first fitting piece side wall 42a is inclined to the radial direction of the tube stent 10 and the first body side wall 41a is flat to the radial direction. Similarly, the second fitting piece side wall 52a of the second fitting hole 53 in the second fitting section 50 is inclined to the radial direction of the outer catheter 20 and the second body side wall 51a is flat to the radial direction of the outer catheter 20. It should be noted that the first coupling section 45 and the second coupling section 55 are similar to those in the first embodiment.

The respective fitting sections thus configured allow load distribution for pushing the tube stent 10 using the outer catheter because the first body side wall 41a is flat to the radial direction and the second body side wall 51a is flat to the radial direction of the outer catheter 20. As a result, it is possible to push the tube stent 10 using the outer catheter 20 without deforming the end portions of the tube stent 10 and the outer catheter 20.

In the third embodiment of the fitting sections illustrated in Figs. 7D, 7E, and 7F, the first fitting piece side wall 42a and the first body side wall 41a are flat to the radial direction of the tube stent 10. Similarly, the second fitting piece side wall 52a and the second body side wall 51a are flat to the radial direction of the outer catheter 20. The first coupling section 45 and the second coupling section 55 are parallel to the longitudinal direction (axial direction) of the tube stent 10 and the outer catheter 20. As just described, in the present embodiment, the first fitting piece side wall 42a and the first body side wall 41a have shapes symmetrical (same shape) about the first coupling section 45 and the second fitting piece side wall 52a and the second body side wall 51a have shapes symmetrical (same shape) about the second coupling section 55.

The respective fitting sections thus configured allow load distribution for pushing or pulling the tube stent 10 using the outer catheter 20 because the first fitting piece side wall 42a and the first body side wall 41a are flat to the radial direction and the second fitting piece side wall 52a and the second body side wall 51a are flat to the radial direction of the outer catheter 20. As a result, it is possible to push and pull the tube stent 10 using the outer catheter 20 without deforming the end portions of the tube stent 10 and the outer catheter 20.

In the fourth embodiment of the fitting sections illustrated in Figs. 8A, 8B, and 8C, the first fitting piece side wall 42a and the first body side wall 41a are flat to the radial direction of the tube stent 10 similar to the third embodiment while the first coupling section 45 is formed longer in the longitudinal direction (axial direction) of the tube stent 10 compared with that in the third embodiment. The second fitting piece side wall 52a and the second body side wall 51a are flat to the radial direction of the outer catheter 20 while the second coupling section 55 is formed longer in the longitudinal direction (axial direction) of the outer catheter 20 compared with that in the third embodiment.

The respective fitting sections thus configured allow load distribution for pushing or pulling the tube stent 10 using the outer catheter 20 because the first fitting piece side wall 42a and the first body side wall 41a are flat to the radial direction and the second fitting piece side wall 52a and the second body side wall 51a are flat to the radial direction of the outer catheter 20. As a result, it is possible to push and pull the tube stent 10 using the outer catheter 20 without deforming the end portions of the tube stent 10 and the outer catheter 20.

In the fifth embodiment of the fitting sections illustrated in Figs. 8D, 8E, and 8F, the first fitting piece side wall 42a and the first body side wall 41a are inclined to the radial direction of the tube stent 10 and the first fitting hole 43 is formed in a substantially triangular shape in a side view. Similarly, the second fitting piece side wall 52a and the second body side wall 51a are inclined to the radial direction of the outer catheter 20 and the second fitting hole 53 is formed in a substantially triangular shape in a side view. In this situation, the first coupling section 45 and the second coupling section 55 are in the vertex positions of the substantially triangular shapes.

The respective fitting sections thus configured also allow obtaining the same effects as those in the first embodiment. Since the first body side wall 41a and the second fitting piece side wall are inclined to the radial direction, pulling back the outer catheter 20 causes natural shifting of the axis of the tube stent 10 from the axis of the outer catheter 20. As a result, it is possible to readily separate the first fitting section 40 from the second fitting section 50.

In the sixth embodiment of the fitting sections illustrated in Figs. 9A, 9B, and 9C, the first fitting piece side wall 42a, the first body side wall 41a, and the first coupling section 45 have curved surface shapes (curved shapes) and the first fitting hole 43 as a whole is formed in an arc or U-shape in a side view. Similarly, the second fitting piece side wall 52a, the second body side wall 51a, and the second coupling section 55 have curved surface shapes (curved shapes) and the second fitting hole 53 as a whole is formed in an arc or U-shape in a side view.

As just described, in the present embodiment, the first fitting piece side wall 42a, the first body side wall 41a, and the first coupling section 45 are curved in a side view and the second fitting piece side wall 52a, the second body side wall 51a, and the second coupling section 55 are curved in a side view.

The respective fitting sections thus configured allow, in addition to obtaining the same effects as those in the first embodiment, more load distribution for pushing or pulling the tube stent 10 using the outer catheter 20 because the first fitting piece side wall 42a, the first body side wall 41a, the first coupling section 45, the second fitting piece side wall 52a, the second body side wall 51a, and the second coupling section 55 have curved surface shapes (curved shapes). As a result, it is possible to push and pull the tube stent 10 using the outer catheter 20 without deforming the end portions of the tube stent 10 and the outer catheter 20. The fitting sections according to the present embodiment may further be readily formed using a cylindrically punching machine or the like.

In the seventh embodiment of the fitting sections illustrated in Figs. 9D, 9E, and 9F, similar to the sixth embodiment, the first fitting hole 43 is formed in an arc or U-shape in a side view. However, an area of the first tube wall section 11 facing the first fitting hole 43 is further provided with a third notch portion 46 formed by partially notching the area. As a result, a pair of first coupling sections 45 integrally connect the first fitting piece 42 and the first body 41.

Similar to the sixth embodiment, the second fitting hole 53 is formed in an arc or U-shape in a side view. However, an area of the second tube wall section 21 facing the second fitting hole 53 is further provided with a fourth notch portion 56 formed by partially notching the area. As a result, a pair of second coupling sections 55 integrally connect the second fitting piece 52 and the second body 51. In this situation, the third notch portion 46 and the fourth notch portion 56 may have a shape employed as the shape of the fitting sections described in the first through sixth embodiments.

That is, in the first fitting section 40 of the seventh embodiment, the first notch portion 44 and the third notch portion 46 are in facing positions and the pair of first coupling sections 45 integrally linking the first fitting piece 42 to the first body 41 are provided between the first notch portion 44 and the third notch portion 46. Similarly, in the second fitting section 50, the second notch portion 54 and the fourth notch portion 56 are in facing positions and the pair of second coupling sections 55 integrally linking the second fitting piece 52 to the second body 51 are provided between the second notch portion 54 and the fourth notch portion 56.

The respective fitting sections thus configured allow, in addition to obtaining the same effects as those in the sixth embodiment, more reduction in diameter of the fitting sections for fitting the tube stent 10 to the outer catheter 20.

In the eighth embodiment illustrated in Figs. 10A, 10B, and 10C, similar to the sixth embodiment, the second fitting hole 53 is formed in an arc or U-shape in a side view. However, the first fitting hole 43 has a smaller size compared with the size of the second fitting hole 53.

The respective fitting sections thus configured allow, in addition to obtaining the same effects as those in the sixth embodiment, an increase in rigidity of the tube stent 10 and improvement in strength of the tube stent 10.

The ninth embodiment illustrated in Figs. 10D, 10E, and 10F is a combination of the sixth embodiment and the seventh embodiment. In the ninth embodiment, the first fitting section 40 is that in the sixth embodiment and the second fitting section 50 is that in the seventh embodiment. The first fitting section 40 and the second fitting section 50 until the seventh embodiment have an identical or similar shape, whereas the first fitting section 40 and the second fitting section 50 in the ninth embodiment have different shapes.

The respective fitting sections thus configured also allow, in addition to obtaining the same effects as those in the sixth embodiment, more reduction in diameter in the direction of forming the notch portion 56 of the fitting sections for fitting the tube stent 10 to the outer catheter 20.

The first fitting section 40 in the tenth embodiment illustrated in Fig. 11 has a component member with a greater thickness compared with that in the first fitting section in the ninth embodiment. The first body has flap sections 13a and 13b provided in an upper portion and a lower portion (near the first fitting hole 43 side of the first body) of the surface and no hole is formed in the first body facing the flap sections 13a and 13b. It should be noted that the flap sections 13a and 13b have a shape that is not particularly limited. In this situation, "near the first fitting hole side of the first body" is not particularly limited as long as effects described later (increase in rigidity, etc.) are obtained in the position (location).

The first fitting section 40 thus configured allows the first fitting section 40 in the present embodiment to have greater rigidity than that of the first fitting section in the embodiments described above to prevent rupture and breakage of the tube stent 10 even when the first fitting section 40 of the tube stent 10 or a nearby area is grasped with forceps for removing the tube stent 10. As a result, it is possible to readily and securely remove the tube stent 10. In particular, flap sections used to be conventionally formed by cutting into part of the tube stent and heating and bending the area (e.g., refer to the flap sections 13 in Figs. 2), whereas the flap sections 13a and 13b in the present embodiment are formed without having to cutting into the tube stent. As a result, the tube stent 10 in the present embodiment has greater rigidity compared with that in the conventional tube stent.

It should be noted that the first fitting section 40 in the present embodiment may be prepared as follows. For example, an external tube with an inner diameter greater than the outer profile of the tube stent in the ninth embodiment is prepared to form a pair of projecting areas to be the flap sections 13a and 13b at an end portion. Then, into the tube, the tube stent in the ninth embodiment is inserted. They are then heated to heat seal the tube stent and the external tube and also to bend the projecting portions, thereby forming the flap sections 13a and 13b. Unnecessary portions are then cut off to allow preparation of the tube stent 10 with the first fitting section 40.

It should be noted that, although the first fitting section 40 in the present embodiment is configured to have the flap sections 13a and 13b in the upper portion and the lower portion of the surface of the first body, the present invention is not limited to this configuration. For example, the first fitting section may be configured to have flap sections in either one of the upper portion and the lower portion of the surface of the first body. The first fitting section thus configured also obtains the same effects as the first fitting section 40 in the present embodiment.

While the differences in shape of the first fitting section 40 and the second fitting section 50 have been described in the first through tenth embodiments, the fitting structure includes various shapes and is not limited only to the embodiments described above.

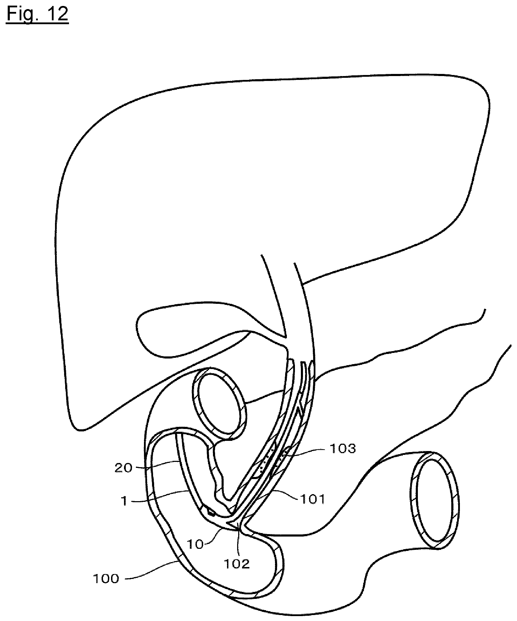

Based on Fig. 12, a description is then given to a state of using the tube stent delivery system 1 of the present invention in the body. The tube stent delivery system 1 of the present invention is capable of delivering the tube stent 10 to a target site in the body by being used together with an endoscope.

For example, the tube stent 10 is inserted from the duodenum 100 into the papilla 102 of a bile duct 101 and carried to a strictured area 103 in the bile duct. Although the duodenum 100 and the bile duct 101 are substantially orthogonal, the tube stent delivery system 1 of the present invention allows smooth delivery of the tube stent 10 to the target site without separating the fitting area between the tube stent 10 and the outer catheter 20 due to the tight fitting between the first fitting section 40 and the second fitting section 50.

The inner catheter 30 is then withdrawn at the time when the tube stent 10 is arranged at the target site and biliary drainage is successfully performed, and the axis of the tube stent 10 is shifted from the axis of the outer catheter 20, thereby allowing smooth separation of the first fitting section 40 from the second fitting section 50.

### Simulation

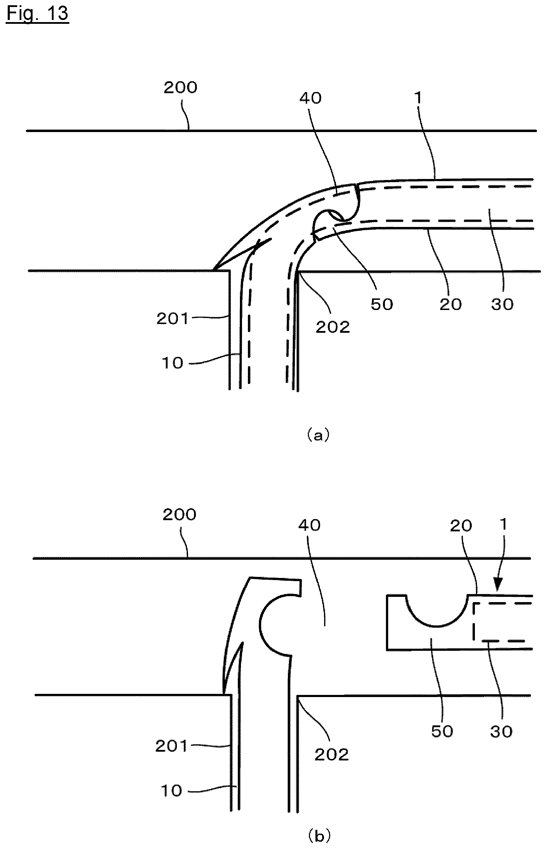

Based on Figs. 13, a simulation of the tube stent delivery system 1 of the present invention is described. The present inventor performed a simulation to confirm the efficacy of the tube stent delivery system 1 of the present invention.

At first, a model is prepared with a thick pipe 200 representing the duodenum 100 and a narrow pipe 201 representing the bile duct 101 and orthogonally connected to the thick pipe 200. The tube stent 10 inserted from the thick pipe 200 is inserted into the narrow pipe 201 while being guided by the inner catheter 30 (broken lines in the drawing). Deformation of the junction of the tube stent 10 with the outer catheter 20 is then observed near a perpendicularly bending area 202 representing the papilla. As a result, they are deformed in a curve as if they were one tube.

The connection with a thread described in PTL 1 sometimes causes bending at either distal end portion in in the connecting area of the tube stent and the outer catheter and the thread to cut into the tube stent as described above. However, in the tube stent delivery system 1 of the present invention, it is confirmed that the tube stent and the outer catheter form one continuous tube shape and such an incident does not occur.

Then, the operation of withdrawing the inner catheter 30 is performed. When the distal end of the inner catheter 30 is pulled out of the first insertion section 22 and the fitting area between the first fitting section 40 and the second fitting section 50, the first fitting section 40 and the second fitting section 50 are naturally separated and smooth separation between them is confirmed.

It is confirmed that, compared with the inner catheter having an area with a partially greater diameter described in PTL 2, the inner catheter 30 according to the present invention is withdrawn almost without resistance and smoothly withdrawn. It is thus confirmed that an excessive force is not applied to the tube stent delivery system 1 of the present invention, no position shift does not occur at the distal end of the tube stent 10, and the tube stent 10 is allowed to be readily arranged at the target site. Particularly, it is confirmed that a pigtail tube stent 10 with a distal end portion curved in advance is also allowed to readily separate the first fitting section 40 from the second fitting section 50.

As a result of the present simulation, the following advantages are found.

(1) Movement in the endoscope for adjustment of the indwelling position of the tube stent 10 does not cause easy disconnection of the tube stent 10 as the tube stent delivery system described in PTL 1.
(2) Even in a bending junction, which becomes a problem in the tube stent delivery system using a thread as described in PTL 1, it is possible to readily separate the fitting area of the present invention.
(3) Since a gap to pass a thread does not have to be provided between the inner catheter and the outer catheter as in the tube stent delivery system using a thread described in PTL 1, the tube stent delivery system 1 of the present invention is allowed to have a narrower diameter. In particular, it is possible to manufacture a tube stent delivery system with an outer diameter of 1.67 mm (5 French gauge) or less, which used not to be achieved by the conventional technique.
(4) No defect occurs due to a thread cutting into the tube stent or the like as in the tube stent delivery system using a thread described in the conventional tube stent delivery system.
(5) The fitting area of the present invention does not cause a defect of separation (release) in the junction area due to aging, adjustment failure, and the like in the tube stent delivery system as described in PTL 2, examples of the defect including not capable of separating the tube stent, unintentional separation of the tube stent, and the like.
(6) When the tube stent delivery system of the present invention is arranged in an endoscope, the entire circumference of the tube stent delivery system is surrounded by the inner wall of the endoscope, thereby not allowing separation of the tube stent even while the inner catheter is withdrawn.

A detailed description has been given to the examples of the tube stent delivery system 1 of the present invention. The present invention allows fitting of the fitting sections 40 and 50 to each other and the fitting to be firmer by inserting the inner catheter 30 into the insertion sections 12 and 22, thereby allowing prevention of separating the tube stent 10 from the outer catheter 20. As a result, it is possible to stably deliver the tube stent 10 at a target site in the body. In addition, it is possible to readily separate the tube stent 10 from the outer catheter 20 by pulling out the inner catheter 30 of the insertion sections 12 and 22.

In the present invention, the fitting area between the first fitting section 40 and the second fitting section 50 have an overlapping double structure and thus the fitting becomes firmer.

Further in the present invention, the first fitting piece 42 of the first fitting section 40 is inserted into the second fitting hole 53 of the second fitting section 50 and also the second fitting piece 52 of the second fitting section 50 is inserted into the first fitting hole 43 of the first fitting section 40, thereby allowing accurate determination of a relative position of the fitting of the first fitting section 40 and the second fitting section 50 to achieve secure fitting.

Then, the first fitting piece 42 is located at the distal end portion of the tube stent 10 and the second fitting piece 52 is located at the distal end portion of the outer catheter 20, and thus the inner catheter 30 is inserted from the first fitting piece 42 to stabilize the fitting of the first fitting section 40 and the second fitting section 50 and then inserted into the second fitting piece 52, thereby allowing achievement of tighter fitting.

The state of inserting the inner catheter 30 into the respective insertion sections 12 and 22 while the first fitting piece 42 is inserted into the second fitting hole 53 and the second fitting piece 52 is inserted into the first fitting hole 43 is a state in which the inner catheter 30 is inserted into both the first body 41 and the second body 51, thereby causing the combination of the tube stent 10 with the outer catheter 20 firmer and reliable delivery of the tube stent 10 to a target site in the body without easily separated. Moreover, the fitting of the first fitting section 40 and the second fitting section 50 is readily separated only by withdrawing the inner catheter 30, and it is thus possible to indwell the tube stent 10 in a desired position with no load on the tube stent 10 reaching the target site.

The tube stent 10 and the outer catheter 20 are partially notched to allow readily providing the notch portions 44 and 45 and the coupling sections 44 and 45. As a result, it is possible to precisely form the fitting sections 40 and 50 in the tube stent 10 and the outer catheter 20.

Since the fitting holes 43 and 53 are configured with the fitting piece side walls 42a and 52a, the body side walls 41a and 51a, and the coupling sections 44 and 45, the fitting is securely made by the respective abutment during the fitting. Different from the conventional tube stent delivery system described above, the abutment at a plurality of contact points allows preventing the rear end portion of the tube stent from cutting into the distal end portion of the outer catheter even when the fitting area is bending, and thus the fitting area is readily separated and the load of withdrawing the inner catheter 30 is reduced.

When the first fitting section 40 and the second fitting section 50 have an identical or similar shape, the fitting is stabilized.

When the facing side walls (41a and 42a, and 51a and 52a) of the fitting holes 43 and 53 have the same shape (shapes symmetrical about the first coupling section 45 in a side view and shapes symmetrical about the second coupling section 55 in a side view) for the fitting, the fitting of the tube stent 10 and the outer catheter 20 is stabilized and displacement of the tube stent 10 relative to the outer catheter 20 while moving in the body is reduced.

When the first fitting section 40 and the second fitting section 50 have different shapes, either one of the fitting sections having a less amount of deformation causes an increase in the amount of deforming the other fitting section, thereby allowing an increase in flexibility.

When the facing side walls (41a and 42a, and 51a and 52a) of the fitting holes 43 and 53 have the same shape (shapes symmetrical about the first coupling section 45 in a side view and shapes symmetrical about the second coupling section 55 in a side view), the loads to push out and pull back the tube stent 10 are same and thus the fitting is stabilized and the displacement while moving in the body is reduced.

When the facing side walls (41a and 42a, and 51a and 52a) of the fitting holes 43 and 53 have different shapes (shapes asymmetrical about the first coupling section 45 in a side view and shapes asymmetrical about the second coupling section 55 in a side view), the load for deformation is distributed and thus the load while moving in the body is reduced.

It should be noted that the present invention is not limited to the embodiments described above and may be appropriately subjected to modification, improvement, and the like. In addition, the materials, the shapes, the dimensions, the numerical values, the modes, the numbers, the arranged areas, and the like of the respective components in the embodiments described above are arbitrary and not limited.

### Other Embodiments

Although the opening direction of the first fitting hole and the opening direction of the second fitting hole are not particularly limited in the embodiments described above, when the tube stent or the outer catheter bends upon withdrawal of the inner catheter from the tube stent or the outer catheter (the tube stent or the outer catheter is in a bending shape in advance), the first fitting hole and the second fitting hole may be formed to open the first fitting hole and the second fitting hole in a direction other than the bending direction.

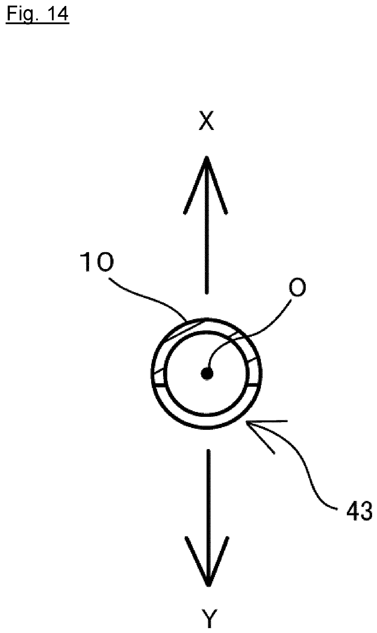

For example, as illustrated in Fig. 14, the first fitting hole 43 may be formed in a direction rotated 180° about the axis of the tube stent relative to a bending direction X of the tube stent 10. It should be noted that Fig. 14 is a cross-sectional view along B-B illustrated in Fig. 2.

The tube stent delivery system thus configured causes the first fitting section of the tube stent to be bent in the X direction by withdrawing the tube stent from the inner catheter, thereby naturally shifting the axis of the tube stent from the axis of the outer catheter. As a result, the first fitting section is naturally separated from the second fitting section.

Similarly, the tube stent delivery system configured to have the second fitting hole opening in a direction other than the bending direction of the outer catheter relative to the bending direction of the outer catheter causes the second fitting section of the outer catheter to be bent by withdrawing the inner catheter from the outer catheter, thereby naturally shifting the axis of the tube stent from the axis of the outer catheter. As a result, the first fitting section is naturally separated from the second fitting section.

In this situation, although the opening direction of the first fitting hole is not particularly limited as long as it is a direction other than the bending direction of the tube stent, the direction preferably ranges from 90° to 270° about the axis of the tube stent (the rotating direction is not particularly limited) relative to the bending direction of the tube stent because the first fitting section 40 and the second fitting section 50 are readily separated, more preferably ranges from 120° to 240° because the first fitting section 40 and the second fitting section 50 are more readily separated, and particularly preferably ranges from 150° to 210° because the first fitting section 40 and the second fitting section 50 are even more readily separated. Such relationship also applies to the relationship between opening direction of the second fitting hole and the bending direction of the outer catheter.

It should be noted that, although the present embodiment describes the cases where the tube stent and the outer catheter are bent due to the properties (internal factors), such as the shape, of the tube stent and the outer catheter themselves, the present invention is not limited to such a case. For example, the present invention naturally includes the cases where the tube stent and the outer catheter are bent due to an external factor, such as the shape of a location in which the tube stent or the outer catheter is arranged.

Although only one first fitting section and one second fitting section are formed respectively in the tube stent and the outer catheter in the embodiments described above, a plurality of them may be naturally formed.

Although the positions of the first fitting section and the second fitting section are not particularly limited in the embodiments described above, they may be formed in the following positions. The first fitting section is preferably formed to be located in a range from 2 mm to 55 mm from the end portion of the tube stent because it is possible to readily fit the first fitting section and the second fitting section and also readily remove the fitting with the second fitting section by readily shifting the axis of the tube stent from the axis of the outer catheter. The first fitting section is more preferably formed to be located in a range from 2 mm to 30 mm from the end portion of the tube stent because it is possible to more readily fit the first fitting section and the second fitting section and also more readily remove the fitting with the second fitting section by more readily shifting the axis of the tube stent from the axis of the outer catheter. The first fitting section is particularly preferably formed to be located in a range from 2 mm to 10 mm from the end portion of the tube stent because it is possible to even more readily fit the first fitting section and the second fitting section and also even more readily remove the fitting with the second fitting section by even more readily shifting the axis of the tube stent from the axis of the outer catheter.

Similarly, the second fitting section is preferably formed to be located in a range from 2 mm to 55 mm from the end portion of the outer catheter because it is possible to readily fit the second fitting section and the first fitting section and also readily remove the fitting with the first fitting section by readily shifting the axis of the tube stent from the axis of the outer catheter. The second fitting section is more preferably formed to be located in a range from 2 mm to 30 mm from the end portion of the outer catheter because it is possible to more readily fit the second fitting section and the first fitting section and also more readily remove the fitting with the first fitting section by more readily shifting the axis of the tube stent from the axis of the outer catheter. The second fitting section is particularly preferably formed to be located in a range from 2 mm to 10 mm from the end portion of the outer catheter because it is possible to even more readily fit the second fitting section and the first fitting section and also even more readily remove the fitting with the first fitting section by even more readily shifting the axis of the tube stent from the axis of the outer catheter.

In this situation, the position of the first fitting section means a distance from the end portion of the tube stent to the end portion on the opposite side of the end portion in the first fitting section of the tube stent. For example, in the first embodiment, the first fitting section located at 10 mm from the end portion of the tube stent 10 means that the distance between the end portion of the tube stent 10 and the position of the first body side wall 41a most away from the end portion of the tube stent 10 is 10 mm (refer to L in Fig. 5A). The same applies to the position of the second fitting section.

### Industrial Applicability

The stent delivery system of the present invention is most appropriate in the fields expected to readily indwell a tube stent at a target site in the body.

### Reference Signs List

- 1: Stent Delivery System
- 10: Tube Stent
- 11: First Tube Wall Section
- 12: First Insertion Section
- 13, 13a, 13b: Flap Section
- 20: Outer Catheter
- 21: Second Tube Wall Section
- 22: Second Insertion Section
- 23: Inner Connector
- 30, 130: Inner Catheter
- 31: Outer Connector
- 40: First Fitting Section
- 41: First Body
- 41a: First Body Side Wall
- 42: First Fitting Piece
- 42a: First Fitting Piece Side Wall
- 43: First Fitting Hole
- 44: First Notch Portion
- 45: First Coupling Section
- 46: Third Notch Portion
- 50: Second Fitting Section
- 51: Second Body
- 51a: Second Body Side Wall
- 52: Second Fitting Piece
- 52a: Second Fitting Piece Side Wall
- 53: Second Fitting Hole
- 54: Second Notch Portion
- 55: Second Coupling Section
- 56: Fourth Notch Portion
- O: Axis of Tube Stent
- X: Bending Direction of Tube Stent
- Y: Opening Direction of First Fitting Hole

## Claims

1. A tube stent delivery system (1), comprising: a tube stent (10); an outer catheter (20); and an inner catheter (30, 130), wherein
the tube stent (10) has a first tube wall section (11) in an annular shape, a hollow first insertion section (12) surrounded by the first tube wall section (11), and a first fitting section (40) fitted to the outer catheter (20),
the first fitting section (40) has a first fitting piece (42), a first fitting hole (43), and a first body (41),
the outer catheter (20) has a second tube wall section (21) in an annular shape, a hollow second insertion section (22) surrounded by the second tube wall section (21), and a second fitting section (50) fitted to the tube stent (10),
the second fitting section (50) has a second fitting piece (52), a second fitting hole (53), and a second body (51),
the first fitting piece (42) is inserted into the second fitting hole (53) and the second fitting piece (52) is inserted into the first fitting hole (43), thereby performing the fitting between the first fitting section (40) and the second fitting section (50),
the inner catheter (30, 130) is inserted into the first insertion section (12) of the tube stent (10) and the second insertion section (22) of the outer catheter (20), thereby maintaining the fitting,
such that when the inner catheter (30, 130) is withdrawn from at least the first insertion section (12) and the first fitting section (40) of the tube stent (10), the fitting is separated.

2. The tube stent delivery system (1) according to Claim 1, wherein
the first fitting piece (42) has the first insertion section (12) in a tubular shape,
the second fitting piece (52) has the second insertion section (22) in a tubular shape,
the first fitting piece (42) is located at a distal end portion of the tube stent (10), and
the second fitting piece (52) is located at a distal end portion of the outer catheter (20).

3. The tube stent delivery system (1) according to Claim 1 or 2, wherein
the tube stent (10) has a shape bending in a first predetermined direction (X),
the first fitting hole (43) opens in a direction (Y) other than the first predetermined direction (X) of the tube stent (10), and
when the inner catheter (30, 130) is withdrawn from at least the first insertion section (12) and the first fitting section (40) of the tube stent (10), the tube stent (10) is bent in the first predetermined direction (X) to separate the fitting.

4. The tube stent delivery system (1) according to Claim 3, wherein the first fitting hole (43) opens in a direction (Y) ranging from 90° to 270° about an axis of the tube stent (10) relative to the first predetermined direction (X).

5. The tube stent delivery system (1) according to any one of Claims 1 through 4, wherein
the outer catheter (20) has a shape bending in a second predetermined direction,
the second fitting hole (53) opens in a direction other than the second predetermined direction of the outer catheter (20), and
when the inner catheter (30, 130) is withdrawn from the second insertion section (22) and the second fitting section (50) of the outer catheter (20), the outer catheter (20) is bent in the second predetermined direction to separate the fitting.

6. The tube stent delivery system (1) according to Claim 5, wherein the second fitting hole (53) opens in a direction ranging from 90° to 270° about an axis of the outer catheter (20) relative to the second predetermined direction.

7. The tube stent delivery system (1) according to any one of Claims 1 through 6, wherein the first fitting section (40) and the second fitting section (50) have an identical or similar shape.

8. The tube stent delivery system (1) according to any one of Claims 1 through 6, wherein the first fitting section (40) and the second fitting section (50) have different shapes.

9. The tube stent delivery system (1) according to any one of Claims 1 through 6, wherein
the first fitting hole (43) includes
a first fitting piece side wall (42a) provided in the first fitting piece (42),
a first body side wall (41a) of the first body (41) provided in a position facing the first fitting piece side wall (42a), and
a first coupling section (45) integrally linking the first fitting piece side wall (42a) to the first body side wall (41a), and
the second fitting hole (53) includes
a second fitting piece side wall (52a) provided in the second fitting piece (52),
a second body side wall (51a) of the second body (51) provided in a position facing the second fitting piece side wall (52a), and
a second coupling section (55) integrally linking the second fitting piece side wall (52a) to the second body side wall (51a).

10. The tube stent delivery system (1) according to Claim 9, wherein
the first fitting piece side wall (42a) and the first body side wall (41a) have shapes symmetrical about the first coupling section (45) in a side view, and
the second fitting piece side wall (52a) and the second body side wall (51a) have shapes symmetrical about the second coupling section (55) in a side view.

11. The tube stent delivery system (1) according to Claim 9, wherein
the first fitting piece side wall (42a) and the first body side wall (41a) have shapes asymmetrical about the first coupling section (45) in a side view, and
the second fitting piece side wall (52a) and the second body side wall (51a) have shapes asymmetrical about the second coupling section (55) in a side view.

12. The tube stent delivery system (1) according to Claim 9, wherein
the first fitting piece side wall (42a), the first body side wall (41a), and the first coupling section (45) have curved shapes, and
the second fitting piece side wall (52a), the second body side wall (51a), and the second coupling section (55) have curved shapes.

13. The tube stent delivery system (1) according to Claim 9, wherein
the first fitting piece side wall (42a), the first body side wall (41a), and the first coupling section (45) have arc shapes or U shapes, and
the second fitting piece side wall (52a), the second body side wall (51a), and the second coupling section (55) have arc shapes or U shapes.

14. The tube stent delivery system (1) according to any one of Claims 1 through 13, wherein a member forming the first fitting piece (42) and the first fitting hole (43) has a thickness greater than a thickness of a member forming the first body (41).

15. The tube stent delivery system (1) according to any one of Claims 1 through 14, wherein a member forming the second fitting piece (52) and the second fitting hole (53) has a thickness greater than a thickness of a member forming the second body (51).

## Patentansprüche

1. Schlauchstent-Einbringungssystem (1), umfassend: einen Schlauchstent (10); einen äußeren Katheter (20); und einen inneren Katheter (30, 130), wobei
der Schlauchstent (10) einen ersten ringförmigen Schlauchwandabschnitt (11), einen hohlen ersten Einführungsabschnitt (12), der von dem ersten Schlauchwandabschnitt (11) umgeben ist, und einen ersten Anschlussabschnitt (40) aufweist, der an dem äußeren Katheter (20) angebracht ist,
der erste Anschlussabschnitt (40) ein erstes Anschlussstück (42), ein erstes Anschlussloch (43) und einen ersten Körper (41) aufweist,
der äußere Katheter (20) einen zweiten ringförmigen Schlauchwandabschnitt (21), einen hohlen zweiten Einführungsabschnitt (22), der von dem zweiten Schlauchwandabschnitt (21) umgeben ist, und einen zweiten Anschlussabschnitt (50) aufweist, der an dem Schlauchstent (10) angebracht ist,
der zweite Anschlussabschnitt (50) ein zweites Anschlussstück (52), ein zweites Anschlussloch (53) und einen zweiten Körper (51) aufweist,
das erste Anschlussstück (42) in das zweite Anschlussloch (53) und das zweite Anschlussstück (52) in das erste Anschlussloch (43) eingesetzt wird, wodurch der Anschluss zwischen dem ersten Anschlussabschnitt (40) und dem zweiten Anschlussabschnitt (50) erfolgt,
der innere Katheter (30, 130) in den ersten Einführungsabschnitt (12) des Schlauchstents (10) und den zweiten Einführungsabschnitt (22) des äußeren Katheters (20) eingeführt wird, wodurch der Anschluss beibehalten wird,
so dass, wenn der innere Katheter (30, 130) zumindest aus dem ersten Einführungsabschnitt (12) und dem ersten Anschlussabschnitt (40) des Schlauchstents (10) herausgezogen wird, der Anschluss getrennt wird.

2. Schlauchstent-Einbringungssystem (1) nach Anspruch 1, wobei
das erste Anschlussstück (42) den ersten Einführungsabschnitt (12) in einer schlauchförmigen Form aufweist,
das zweite Anschlussstück (52) den zweiten Einführungsabschnitt (22) in einer schlauchförmigen Form aufweist,
das erste Anschlussstück (42) an einem distalen Endabschnitt des Schlauchstents (10) angeordnet ist, und
das zweite Anschlussstück (52) an einem distalen Endabschnitt des äußeren Katheters (20) angeordnet ist.

3. Schlauchstent-Einbringungssystem (1) nach Anspruch 1 oder 2, wobei
der Schlauchstent (10) eine in einer ersten vorbestimmten Richtung (X) gebogene Form aufweist,
das erste Anschlussloch (43) sich in eine andere Richtung (Y) als die erste vorbestimmte Richtung (X) des Schlauchstents (10) öffnet, und
wenn der innere Katheter (30, 130) aus zumindest dem ersten Einführungsabschnitt (12) und dem ersten Anschlussabschnitt (40) des Schlauchstents (10) herausgezogen wird, der Schlauchstent (10) in der ersten vorbestimmten Richtung (X) gebogen wird, um den Anschluss zu trennen.

4. Schlauchstent-Einbringungssystem (1) nach Anspruch 3, wobei sich das erste Anschlussloch (43) in einer Richtung (Y) im Bereich von 90° bis 270° um eine Achse des Schlauchstents (10) relativ zu der ersten vorbestimmten Richtung (X) öffnet.

5. Schlauchstent-Einbringungssystem (1) nach einem der Ansprüche 1 bis 4, wobei
der äußere Katheter (20) eine in einer zweiten vorbestimmten Richtung gebogene Form aufweist,
das zweite Anschlussloch (53) sich in eine andere Richtung als die zweite vorbestimmte Richtung des äußeren Katheters (20) öffnet, und
wenn der innere Katheter (30, 130) aus dem zweiten Einführungsabschnitt (22) und dem zweiten Anschlussabschnitt (50) des äußeren Katheters (20) herausgezogen wird, der äußere Katheter (20) in der zweiten vorbestimmten Richtung gebogen wird, um den Anschluss zu trennen.

6. Schlauchstent-Einbringungssystem (1) nach Anspruch 5, wobei sich das zweite Anschlussloch (53) in einer Richtung im Bereich von 90° bis 270° um eine Achse des äußeren Katheters (20) relativ zu der zweiten vorbestimmten Richtung öffnet.

7. Schlauchstent-Einbringungssystem (1) nach einem der Ansprüche 1 bis 6, wobei der erste Anschlussabschnitt (40) und der zweite Anschlussabschnitt (50) eine identische oder ähnliche Form aufweisen.

8. Schlauchstent-Einbringungssystem (1) nach einem der Ansprüche 1 bis 6, wobei der erste Anschlussabschnitt (40) und der zweite Anschlussabschnitt (50) unterschiedliche Formen aufweisen.

9. Schlauchstent-Einbringungssystem (1) nach einem der Ansprüche 1 bis 6, wobei
das erste Anschlussloch (43) umfasst:
eine erste Anschlusstück-Seitenwand (42a), die in dem ersten Anschlusstück (42) vorgesehen ist,
eine erste Körperseitenwand (41a) des ersten Körpers (41), die in einer Position vorgesehen ist, die der ersten Anschlussstückseitenwand (42a) zugewandt ist, und
einen ersten Kupplungsabschnitt (45), der die erste
Anschlussstückseitenwand (42a) mit der ersten Körperseitenwand (41a) einstückig verbindet, und
das zweite Anschlussstückloch (53) umfasst:
eine zweite Anschlusstück-Seitenwand (52a), die in dem zweiten Anschlussteil (52) vorgesehen ist,
eine zweite Körperseitenwand (51a) des zweiten Körpers (51), die in einer Position vorgesehen ist, die der zweiten Anschlussstückseitenwand (52a) zugewandt ist, und
einen zweiten Kupplungsabschnitt (55), der die zweite Anschlussstückseitenwand (52a) mit der zweiten Körperseitenwand (51a) integral verbindet.

10. Schlauchstent-Einbringungssystem (1) nach Anspruch 9, wobei
die erste Anschlussstückseitenwand (42a) und die erste Körperseitenwand (41a) in einer Seitenansicht symmetrische Formen um den ersten Kupplungsabschnitt (45) aufweisen, und
die zweite Anschlussstückseitenwand (52a) und die zweite Körperseitenwand (51a) in einer Seitenansicht symmetrische Formen um den zweiten Kupplungsabschnitt (55) aufweisen.

11. Schlauchstent-Einbringungssystem (1) nach Anspruch 9, wobei
die erste Anschlussstückseitenwand (42a) und die erste Körperseitenwand (41a) in einer Seitenansicht asymmetrische Formen um den ersten Kupplungsabschnitt (45) aufweisen, und
die zweite Anschlussstückseitenwand (52a) und die zweite Körperseitenwand (51a) in einer Seitenansicht asymmetrische Formen um den zweiten Kupplungsabschnitt (55) aufweisen.

12. Schlauchstent-Einbringungssystem (1) nach Anspruch 9, wobei
die erste Anschlussstückseitenwand (42a), die erste Körperseitenwand (41a) und der erste Kupplungsabschnitt (45) gekrümmte Formen aufweisen, und
die zweite Anschlussstückseitenwand (52a), die zweite Körperseitenwand (51a) und der zweite Kupplungsabschnitt (55) gekrümmte Formen aufweisen.

13. Schlauchstent-Einbringungssystem (1) nach Anspruch 9, wobei
die erste Anschlussstückseitenwand (42a), die erste Körperseitenwand (41a) und der erste Kupplungsabschnitt (45) bogenförmig oder U-förmig sind, und
die zweite Anschlussstückseitenwand (52a), die zweite Körperseitenwand (51a) und der zweite Kupplungsabschnitt (55) bogenförmig oder U-förmig ausgebildet sind.

14. Schlauchstent-Einbringungssystem (1) nach einem der Ansprüche 1 bis 13, wobei ein Element, das das erste Anschlussstück (42) und das erste Anschlussloch (43) bildet, eine Dicke hat, die größer ist als die Dicke eines Elements, das den ersten Körper (41) bildet.

15. Schlauchstent-Einbringungssystem (1) nach einem der Ansprüche 1 bis 14, wobei ein Element, das das zweite Anschlussstück (52) und das zweite Anschlussloch (53) bildet, eine Dicke aufweist, die größer ist als die Dicke eines Elements, das den zweiten Körper (51) bildet.

## Revendications

1. Système de pose de stent tubulaire (1), comprenant : un stent tubulaire (10) ;
un cathéter externe (20) ; et un cathéter interne (30, 130),
dans lequel
le stent tubulaire (10) présente une première section de paroi tubulaire (11) de forme annulaire, une première section d'insertion creuse (12) entourée par la première section de paroi tubulaire (11), et une première section de liaison (40) reliée au cathéter externe (20),
la première section de liaison (40) présente une première pièce de liaison (42), un premier trou de liaison (43) et un premier corps (41),
le cathéter extérieur (20) présente une deuxième section de paroi tubulaire (21) de forme annulaire, une deuxième section d'insertion creuse (22) entourée par la deuxième section de paroi tubulaire (21), et une deuxième section de liaison (50) reliée au stent tubulaire (10),
la deuxième section de liaison (50) présente une deuxième pièce de liaison (52), un deuxième trou de liaison (53) et un deuxième corps (51),
la première pièce de liaison (42) est insérée dans le deuxième trou de liaison (53), et la deuxième pièce de liaison (52) est insérée dans le premier trou de liaison (43), réalisant ainsi la liaison entre la première section de liaison (40) et la deuxième section de liaison (50),
le cathéter interne (30, 130) est inséré dans la première section d'insertion (12) du stent tubulaire (10) et dans la deuxième section d'insertion (22) du cathéter externe (20), maintenant ainsi ladite liaison,
de sorte que lorsque le cathéter interne (30, 130) est retiré au moins de la première section d'insertion (12) et de la première section de liaison (40) du stent tubulaire (10), la liaison est séparée.

2. Système de pose de stent tubulaire (1) selon la revendication 1,
dans lequel
la première pièce de liaison (42) présente la première section d'insertion (12) sous une forme tubulaire,
la deuxième pièce de liaison (52) présente la deuxième section d'insertion (22) sous une forme tubulaire,
la première pièce de liaison (42) est située au niveau d'une portion d'extrémité distale du stent tubulaire (10), et
la deuxième pièce de liaison (52) est située au niveau d'une portion d'extrémité distale du cathéter externe (20).

3. Système de pose de stent tubulaire (1) selon la revendication 1 ou 2,
dans lequel
le stent tubulaire (10) présente une forme se courbant dans une première direction prédéterminée (X),
le premier trou de liaison (43) s'ouvre dans une direction (Y) autre que la première direction prédéterminée (X) du stent tubulaire (10), et
lorsque le cathéter interne (30, 130) est retiré au moins de la première section d'insertion (12) et de la première section de liaison (40) du stent tubulaire (10), le stent tubulaire (10) est courbé dans la première direction prédéterminée (X) pour séparer ladite liaison.

4. Système de pose de stent tubulaire (1) selon la revendication 3,
dans lequel
le premier trou de liaison (43) s'ouvre dans une direction (Y) allant de 90° à 270° autour d'un axe du stent tubulaire (10) par rapport à la première direction prédéterminée (X).

5. Système de pose de stent tubulaire (1) selon l'une des revendications 1 à 4,
dans lequel
le cathéter externe (20) présente une forme se courbant dans une deuxième direction prédéterminée,
le deuxième trou de liaison (53) s'ouvre dans une direction autre que la deuxième direction prédéterminée du cathéter externe (20), et
lorsque le cathéter interne (30, 130) est retiré de la deuxième section d'insertion (22) et de la deuxième section de liaison (50) du cathéter externe (20), le cathéter externe est courbé dans la deuxième direction prédéterminée pour séparer ladite liaison.

6. Système de pose de stent tubulaire (1) selon la revendication 5,
dans lequel
le deuxième trou de liaison (53) s'ouvre dans une direction allant de 90° à 270° autour d'un axe du cathéter externe (20) par rapport à la deuxième direction prédéterminée.

7. Système de pose de stent tubulaire (1) selon l'une des revendications 1 à 6,
dans lequel
la première section de liaison (40) et la deuxième section de liaison (50) ont une forme identique ou similaire.

8. Système de pose de stent tubulaire (1) selon l'une des revendications 1 à 6,
dans lequel
la première section de liaison (40) et la deuxième section de liaison (50) ont des formes différentes.

9. Système de pose de stent tubulaire (1) selon l'une des revendications 1 à 6,
dans lequel
le premier trou de liaison (43) comprend
une première paroi latérale de pièce de liaison (42a) prévue dans la première pièce de liaison (42),
une première paroi latérale de corps (41a) du premier corps (41) prévue dans une position faisant face à la première paroi latérale de pièce de liaison (42a), et
une première section de couplage (45) reliant intégralement la première paroi latérale de pièce de liaison (42a) à la première paroi latérale de corps (41a), et
le deuxième trou de liaison (53) comprend
une deuxième paroi latérale de pièce de liaison (52a) prévue dans la deuxième pièce de liaison (52),
une deuxième paroi latérale de corps (51a) du deuxième corps (51) prévue dans une position faisant face à la deuxième paroi latérale de pièce de liaison (52a), et
une deuxième section de couplage (55) reliant intégralement la deuxième paroi latérale de pièce de liaison (52a) à la deuxième paroi latérale de corps (51a).

10. Système de pose de stent tubulaire (1) selon la revendication 9,
dans lequel
la première paroi latérale de pièce de liaison (42a) et la première paroi latérale de corps (41a) ont des formes symétriques par rapport à la première section de couplage (55) dans une vue latérale, et
la deuxième paroi latérale de pièce de liaison (52a) et la deuxième paroi latérale de corps (51a) ont des formes symétriques par rapport à la deuxième section de couplage (55) dans une vue latérale.

11. Système de pose de stent tubulaire (1) selon la revendication 9,
dans lequel
la première paroi latérale de pièce de liaison (42a) et la première paroi latérale de corps (41a) ont des formes asymétriques par rapport à la première section de couplage (45) dans une vue latérale, et
la deuxième paroi latérale de pièce de liaison (52a) et la deuxième paroi latérale de corps (51a) ont des formes asymétriques par rapport à la deuxième section de couplage (44) dans une vue latérale.

12. Système de pose de stent tubulaire (1) selon la revendication 9,
dans lequel
la première paroi latérale de pièce de liaison (42a), la première paroi latérale de corps (41a) et la première section de couplage (45) ont des formes incurvées, et
la deuxième paroi latérale de pièce de liaison (52a), la deuxième paroi latérale de corps (51a) et la deuxième section de couplage (55) ont des formes incurvées.

13. Système de pose de stent tubulaire (1) selon la revendication 9,
dans lequel
la première paroi latérale de pièce de liaison (42a), la première paroi latérale de corps (41a) et la première section de couplage (45) ont des formes en arc ou des formes en U, et
la deuxième paroi latérale de pièce de liaison (52a), la deuxième paroi latérale de corps (51a) et la deuxième section de couplage (55) ont des formes en arc ou des formes en U.

14. Système de pose de stent tubulaire (1) selon l'une des revendications 1 à 13, dans lequel
un élément formant la première pièce de liaison (42) et le premier trou de liaison (43) présente une épaisseur supérieure à une épaisseur d'un élément formant le premier corps (41).

15. Système de pose de stent tubulaire (1) selon l'une des revendications 1 à 14, dans lequel
un élément formant la deuxième pièce de liaison (52) et le deuxième trou de liaison (53) présente une épaisseur supérieure à l'épaisseur d'un élément formant le deuxième corps (51).
